(19) Europäisches Patentamt
European Patent Office
Office européen des brevets

(11) **EP 4 239 057 A1**

(12) **EUROPEAN PATENT APPLICATION**
published in accordance with Art. 153(4) EPC

(43) Date of publication:
**06.09.2023 Bulletin 2023/36**

(51) International Patent Classification (IPC):
***C12N 5/071*** *(2010.01)*

(21) Application number: **21898553.9**

(52) Cooperative Patent Classification (CPC):
**C12N 5/06**

(22) Date of filing: **23.11.2021**

(86) International application number:
**PCT/KR2021/017263**

(87) International publication number:
**WO 2022/114727 (02.06.2022 Gazette 2022/22)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.11.2020 KR 20200162867**

(71) Applicant: Xcell Therapeutics Inc.
Seoul 08394 (KR)

(72) Inventors:
• LEE, Ui Il
  Seoul 06326 (KR)
• LEE, Joo Youn
  Seoul 07324 (KR)
• KANG, Hong Seok
  Uiwang-si Gyeonggi-do 16044 (KR)
• LEE, Ji Hye
  Siheung-si Gyeonggi-do 14904 (KR)
• LEE, Jeong Eon
  Seoul 05807 (KR)
• KANG, Min Hee
  Seoul 08354 (KR)

(74) Representative: Isarpatent
Patent- und Rechtsanwälte Barth
Charles Hassa Peckmann & Partner mbB
Friedrichstrasse 31
80801 München (DE)

(54) **CULTURE MEDIUM COMPOSITION FOR ESTABLISHING OR CULTURING KERATINOCYTES COMPRISING CALCIUM, EPIDERAL GROWTH FACTOR, AND ALBUMIN**

(57)  The present invention relates to a culture medium composition for culturing keratinocytes, and more specifically, to a culture medium composition for establishing or culturing keratinocytes, comprising calcium, an epidermal growth factor, and albumin. According to the present invention, when keratinocytes are cultured using the culture medium of the present invention, the keratinocytes can be effectively proliferated within a short time, thus making it possible to more quickly obtain a desired number of the cells during the development of a therapeutic agent using the keratinocytes, and the activity of the keratinocytes is improved, and thus the culture medium of the present invention can be useful in the fields of keratinocyte research and the development of therapeutic agents.

【FIG. 4b】

EP 4 239 057 A1

**Description**

**[TECHNICAL FIELD]**

The present invention relates to a culture medium composition for culturing keratinocytes, and more specifically, to a culture medium composition for establishing or culturing keratinocytes, comprising calcium, an epidermal growth factor and albumin.

**[BACKGROUND ART]**

Keratinocytes present in the basal layer of the epidermis begin to differentiate while migrating upward after division. Such keratinocyte differentiation results in finely regulated changes in gene expression, which is accompanied by cell cycle arrest and massive changes in cell morphology. At this time, representative genes whose expression increases in association to differentiation include transglutaminase, involucrin, cornifin, loricrin, filaggrin, and small proline-rich protein (SPR). Calcium, vitamin D, retinoic acid, 12-o-tetradecanoylphorbol-13-acetate (TPA), etc. are known as factors that affect the differentiation of keratinocytes (Expert Rev Endocrinol Metab. 2012 Jul; 7(4): 461-472).

Among them, calcium is best known as a biological factor that promotes keratinocyte differentiation. In particular, it is known that the calcium concentration increases as it get to upper side of the epidermis. When keratinocyte differentiation proceeds, structural proteins such as involucrin, cornifin, loricrin, and SPR are coupled with transglutamiase on the inside of the cell membrane to form a cornified cell envelope, which is a water-insoluble rigid structure. In particular, calcium is one of the essential elements in cell culture as it is involved in cell survival and metabolic activities, regulates the necessary energy and is involved in the proliferation and differentiation of cells. In the case of conventional keratinocyte media, the calcium concentrations have been used variously in the range from 0.03 mM to 0.1 mM, which may be fatal in research and product development using keratinocytes.

Epidermal growth factor (EGF) was discovered by Dr. Stanley Cohen, an American biologist, and is known as a type of protein that binds to receptors on the surface of the skin and promotes the growth of new cells. Actually, in the case of epidermal growth factor, it is used in many studies and products that culture keratinocytes. This is also used in various concentrations, but according to recent literature, it is known that in the case of high concentrations of epidermal growth factor, transforming growth factor-$\beta$ (TGF-$\beta$) induces a mechanism that inhibits keratinocyte growth.

**[DETAILED DESCRIPTION OF THE INVENTION]**

**[Technical Problem]**

Under such a background, the present inventors have produced a culture medium for culturing keratinocytes, which has a high cell proliferation rate and activity while maintaining the characteristics of keratinocytes, and completed the invention.

Therefore, it is an object of the present invention to provide a culture medium additive for establishing or culturing keratinocytes, comprising calcium, an epidermal growth factor, and albumin.

It is another object of the present invention to provide a culture medium composition for establishing or culturing keratinocytes, comprising the culture medium additive and a basal medium.

It is yet another object of the present invention to provide a method of culturing keratinocytes, comprising culturing keratinocytes in the culture medium composition.

However, the objects of the present invention are not limited to the aforementioned objects, and other objects which are not mentioned herein will be able to be clearly understood by those skilled in the art from the following detailed description.

**[Technical Solution]**

In order to achieve the above object, according to the present invention, there is provided a culture medium additive for establishing or culturing keratinocytes, comprising calcium, an epidermal growth factor, and albumin.

In one embodiment of the present invention, the keratinocytes may be keratinocytes derived from human skin.

In another embodiment of the present invention, the calcium may be contained at a concentration of 0.03 to 0.1 mM.

In yet another embodiment of the present invention, the epidermal growth factor may be contained at a concentration of 0.001 to 1 ng/ml.

In yet another embodiment of the present invention, the albumin may be contained at a concentration of 0.001 mg/ml or more and less than 1 mg/ml.

In still yet another embodiment of the present invention, the culture may be proliferation.

Additionally, according to the present invention, there is provided a culture medium composition for establishing or culturing keratinocytes, comprising the culture medium additive and a basal medium.

In one embodiment of the present invention, the basal medium may be any one selected from the group consisting of DMEM (Dulbecco's Modified Eagle's Medium); MEM (Minimal Essential Medium); BME (Basal Medium Eagle); RPMI 1640; DMEM/F-10 (Dulbecco's Modified Eagle's Medium: Nutrient Mixture F-10); DMEM/F-12 (Dulbecco's Modified Eagle's Medium: Nutrient Mixture F-12); a-MEM (a-Minimal essential Medium); G-MEM (Glasgow's Minimal Essential Medium); IMDM (Isocove's Modified Dulbecco's Medium); KnockOut DMEM (GIBCO, USA); and MCDB.

In another embodiment of the present invention, the culture medium composition may suppress the extension of population doubling time (PDT) of keratinocytes for at least 5 passages.

Further, according to the present invention, there is provided a method of culturing keratinocytes, comprising culturing keratinocytes in the culture medium composition.

[Advantageous Effects]

According to the present invention, when keratinocytes are cultured using the culture medium of the present invention, the keratinocytes can be effectively proliferated within a short time, thus making it possible to more quickly obtain a desired number of the cells during the development of a therapeutic agent using the keratinocytes, and the activity of the keratinocytes is improved, and thus the culture medium of the present invention can be useful in the fields of keratinocyte research and the development of therapeutic agents.

[BRIEF DESCRIPTION OF THE DRAWINGS]

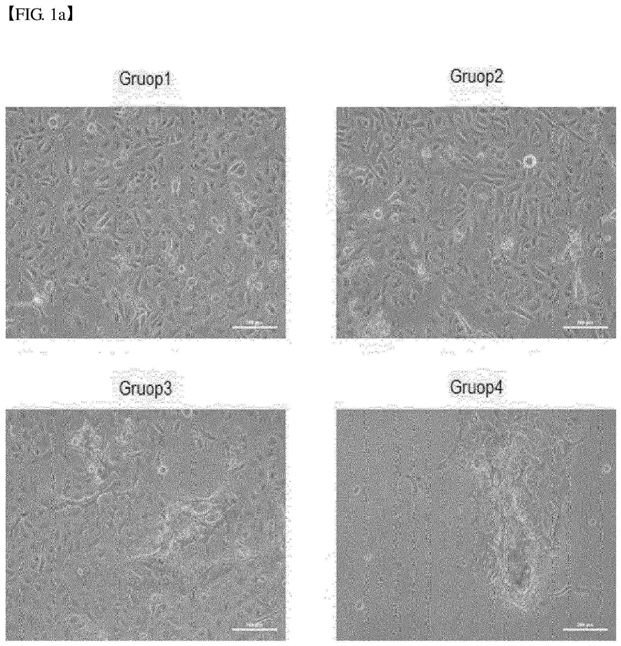

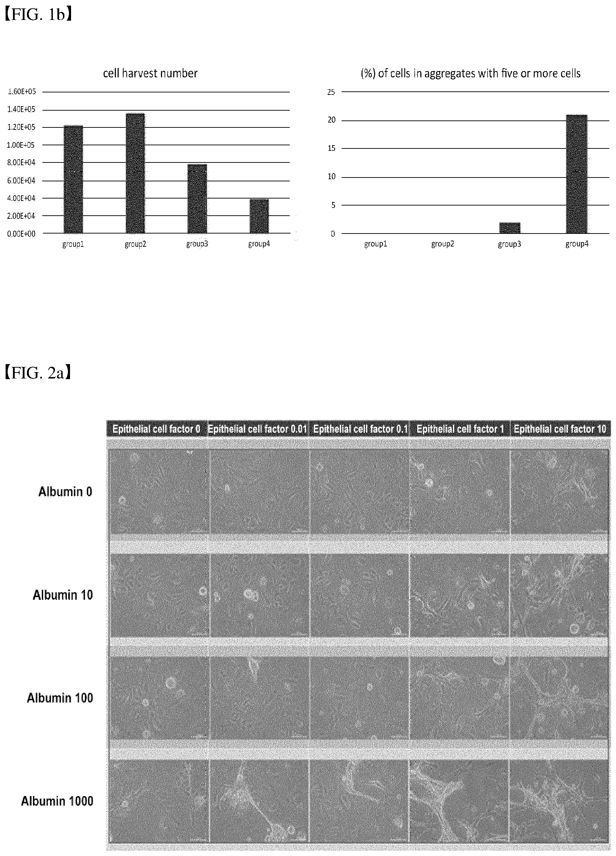

FIG. 1 shows the results of analyzing the effect of calcium concentration on the morphology and proliferation of human keratinocytes. FIG. 1a is a microscope image which shows the cell morphology on the 4th day after culturing keratinocytes in four types of media with different calcium concentrations (0.024, 0.07, 0.14, 0.26 mM), and FIG. 1b shows the results of measuring the number of cells and the degree of aggregation on the 4th day of culture.

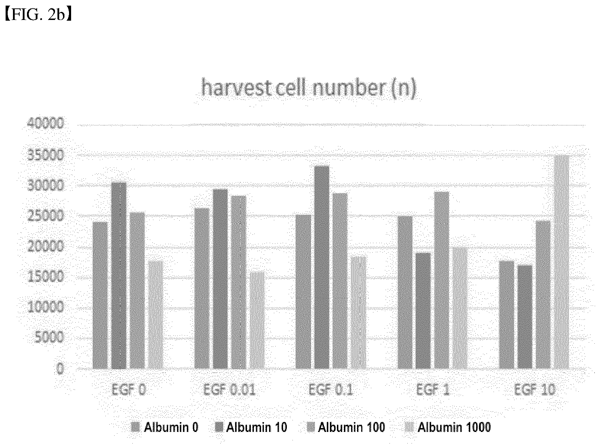

FIG. 2 shows the results of analyzing the effects of epidermal growth factor (EGF) and albumin on the morphology and proliferation of human keratinocytes. FIG. 2a is a microscope image which shows the cell morphology on the 4th day after culturing keratinocytes in a medium containing an epidermal growth factor and albumin respectively different in concentrations as shown in Table 2 below, and FIG. 2b shows the result of measuring the number of cells on the 4th day of culture.

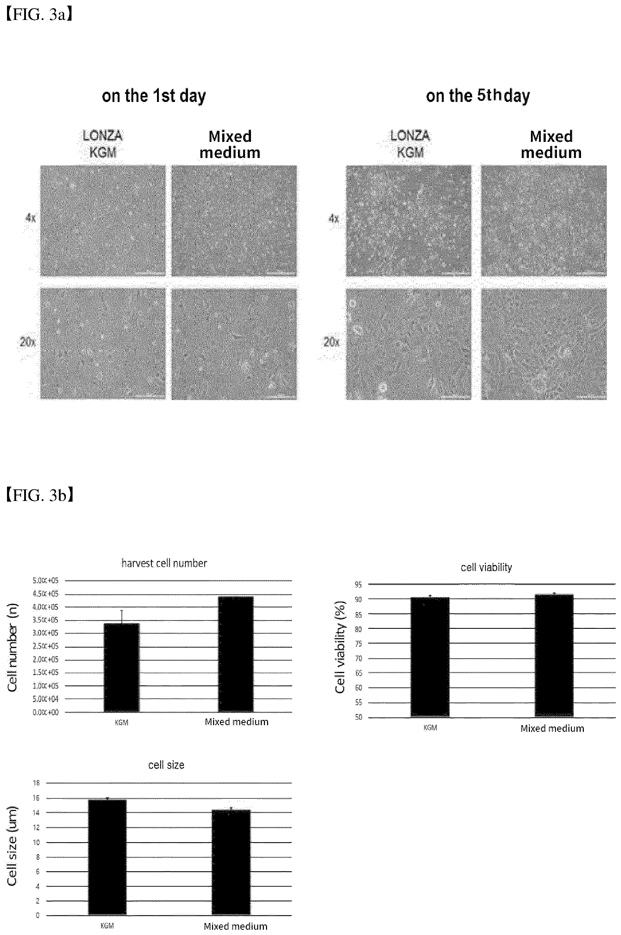

FIG. 3 shows the result of confirming the keratinocyte establishment performance in the mixed medium according to the present invention to which the component ratios of basal medium, calcium, epidermal growth factor (EGF) and albumin are applied. FIG. 3a is a microscopic image of cells cultured in a mixed medium with a commercially available medium (Lonza KGM), which is a positive control group on the 1st day and the 6th day of culture while establishing mouse skin tissue-derived keratinocytes, and FIG. 3b shows the results of measuring the cell number, cell viability, and cell size of the cells cultured in each medium on the 5th day of culture.

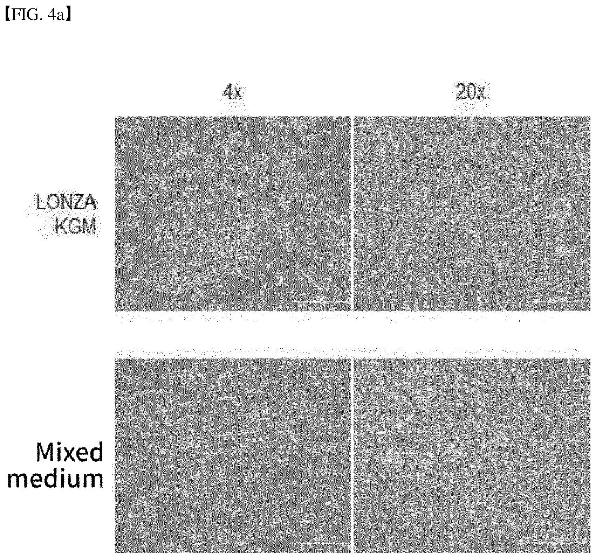

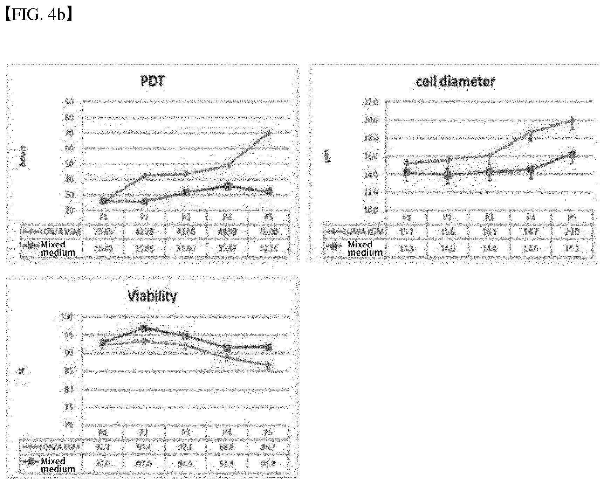

FIG. 4 shows the results of culturing human skin tissue-derived keratinocytes until 5 passages in the mixed medium according to the present invention and a commercially available medium. FIG. 4a shows the microscopic image results of cells (passage 5) cultured in each medium, and FIG. 4b shows the results of measuring the population doubling time (PDT), cell diameter and cell viability of the cells cultured from passage 1 to passage 5.

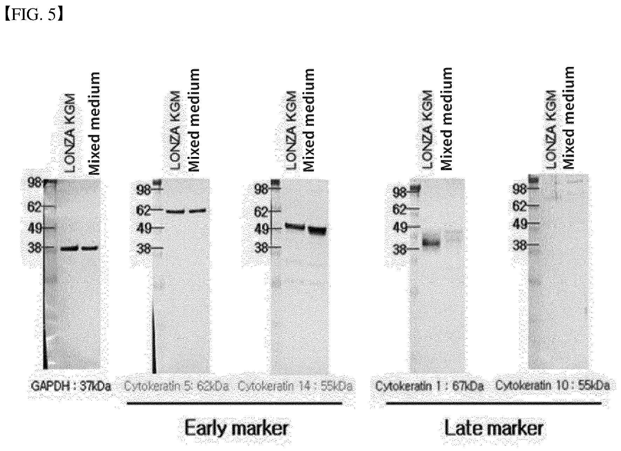

FIG. 5 shows the result of analyzing and comparing the expression levels of keratinocyte-specific marker proteins by performing a Western blot using human keratinocytes (passage 3) cultured in each medium in FIG. 4.

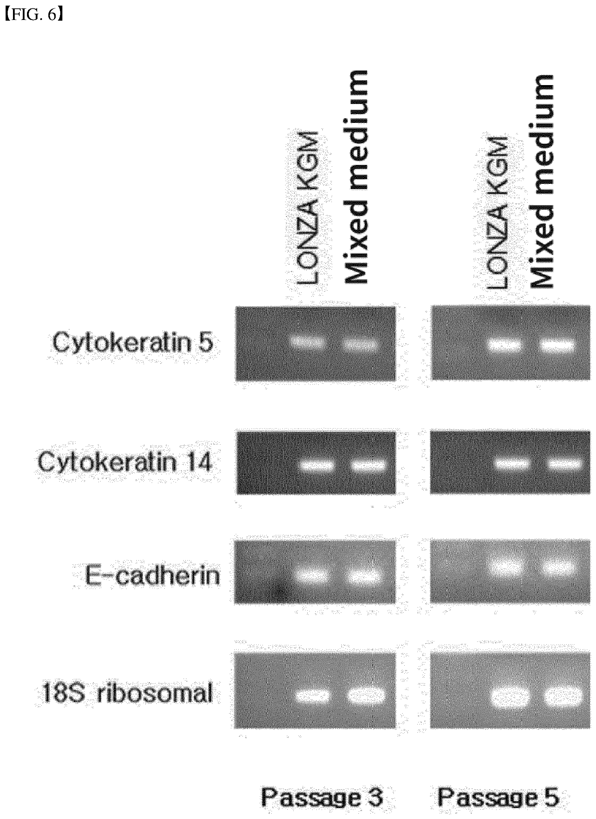

FIG. 6 shows the result of analyzing and comparing the expression level of keratinocyte-specific marker mRNA by performing PCR using human keratinocytes (passage 3 and passage 5) cultured using each medium in FIG. 4 .

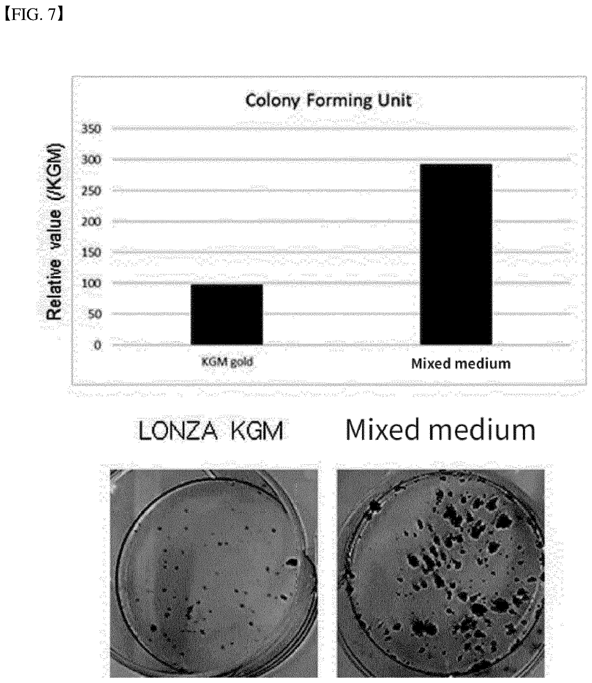

FIG. 7 shows the result of confirming the degree of colony formation by staining human keratinocytes (passage 3) cultured using each medium in FIG. 4 with crystal violet dye.

[DETAILED DESCRIPTION OF THE EMBODIMENTS]

The above objects are achieved from a culture medium additive for establishing or culturing keratinocytes, comprising calcium, an epidermal growth factor, and albumin.

The present invention relates to a culture medium additive for establishing or culturing keratinocytes, which has a high cell proliferation rate and activity while maintaining the characteristics of keratinocytes.

Provided herein is a culture medium additive for establishing or culturing keratinocytes, comprising calcium, an epidermal growth factor, and albumin.

"Keratinocytes", which are the target cells of the present invention, refer to cells of the epidermal and oral epithelial layers that produce keratin in the process of differentiating into the stratum corneum of the skin, and accounts for 95% of skin epidermal cells. In the present invention, the origin of keratinocytes is not particularly limited, but may be preferably derived from humans.

In the present invention, calcium may be contained at a concentration of 0.03 to 0.1 mM, and preferably, it may be contained at a concentration of 0.03 to 0.7mM, without being limited thereto.

In the present invention, the epidermal growth factor may be contained at a concentration of 0.001 to 1 ng/ml, and preferably, it may be contained at a concentration of 0.01 to 0.1 ng/ml, without being limited thereto.

In the present invention, albumin may be contained at a concentration of 0.001 mg/ml or more and less than 1 mg/ml, and preferably, it may be contained at a concentration of 0.01 to 0.1 mg/ml, without being limited thereto.

In the present invention, culturing may be preferably to proliferate keratinocytes.

In another aspect of the present invention, the present invention provides a medium composition for establishing or culturing keratinocytes, comprising the culture medium additive and a basal medium.

In the present invention, the basal medium may be a conventional medium for cell culture, and may contain all components essential for cell culture. The basic medium may be, for example, any one selected from the group consisting of DMEM (Dulbecco's Modified Eagle's Medium); MEM (Minimal Essential Medium); BME (Basal Medium Eagle); RPMI 1640; DMEM/F-10 (Dulbecco's Modified Eagle's Medium: Nutrient Mixture F-10); DMEM/F-12 (Dulbecco's Modified Eagle's Medium: Nutrient Mixture F-12); a-MEM (a-Minimal essential Medium); G-MEM (Glasgow's Minimal Essential Medium); IMDM (Isocove's Modified Dulbecco's Medium); KnockOut DMEM (GIBCO, USA); and MCDB, without being limited thereto.

The present invention analyzes the effects of calcium, epidermal growth factor and albumin on the morphology and proliferation of keratinocytes through specific examples, and derived the optimal concentration effective for proliferation without changing the cell morphology (see Examples 2 and 3).

In another embodiment of the present invention, keratinocytes are cultured using the culture medium composition according to the present invention and an existing commercially available keratinocyte culture medium, and the establishment, proliferation, characteristics, and activity of the cells are analyzed, thereby confirming excellent cell establishment and culture performance of the culture medium of the present invention.

In yet another aspect of the present invention, the present invention provides a method of culturing keratinocytes, comprising culturing keratinocytes in the culture medium composition.

In the present invention, conditions other than the medium composition for culturing keratinocytes, for example, conditions required for culture such as temperature, carbon dioxide concentration, humidity, culture time, culture vessel, etc. are not particularly limited, and can be appropriately selected and applied for efficient culture by those skilled in the art.

Hereinafter, preferred embodiments will be presented in order to help understanding the present invention. However, the following examples are just provided to easily understand the present invention, and the contents of the present invention are not limited thereby.

## [EXAMPLE]

### Example 1. Thawing and culturing of human keratinocytes

The human dermal keratinocytes used in the present invention were transferred under cryopreserved conditions to a cell storage nitrogen tank in a laboratory. After thawing the cryopreserved cells, the supernatant was removed by centrifugation. The cell precipitate was suspended in a commercially available keratinocyte culture medium (LONZA, Gold), inoculated into a T-flask, and then the cells were stabilized in a 37°C incubator while passage 1 proceeded.

### Example 2. Comparison of morphology and proliferation ability of human keratinocytes according to the calcium concentration

Next, the present inventors attempted to investigate the effect of calcium concentration on the morphology and proliferation of human keratinocytes.

For this purpose, the keratinocytes cultured in Example 1 were treated with a trypsin-based enzyme (Gibco™ TrypLE™), and then the cells were harvested and centrifuged at 1000 rpm for 5 minutes. Then, the supernatant was discarded, and the cells were well resuspended by adding the condition-based culture medium shown in Table 1 below, and then the cell numbers were measured using NC-250™ (Chemometec, Denmark). The cells were dispensed at $1 \times 10^4$ cells per well in a 6-well plate, and incubated for 4 days in a 37°C incubator, and a photograph of the cells taken on the 4th day of culture is shown in FIG. 1a. On the 4th day of culture, the cells were treated with a trypsin-based enzyme (Gibco™ TrypLE™), and the cells were harvested and centrifuged at 1000 rpm for 5 minutes. The supernatant of the centrifuged cells was discarded, the cells was well resuspended by adding the condition-based culture medium shown in Table 1 below, and then the cell numbers were measured using NC-250™. When measuring the cell numbers, the degree of cell aggregation was analyzed, and the results are shown in FIG. 1b.

As a result of the experiment, as shown in Table 1, FIGS. 1a and 1b, it was found that if the calcium concentration is higher than 0.1 mM, the cell morphology changes and the recovery rate of the cells decreases. Further, it was confirmed that if the calcium concentration is higher than 0.1 mM, the degree of cell aggregation increases. As a result, it was confirmed that the concentration of calcium is effective between 0.03 and 0.1 mM.

[Table 1]

|  | Group | Detail |
|---|---|---|
| Sample | 1 | 0.024mM calcium |
|  | 2 | 0.07mM calcium |
|  | 3 | 0.14mM calcium |
|  | 4 | 0.26mM calcium |

### Example 3. Comparison of morphology and proliferation ability of human keratinocytes according to the component ratio of epidermal growth factor and albumin

The present inventors attempted to investigate the effects of epidermal growth factor (EGF) and albumin on the morphology and proliferation of human keratinocytes.

For this purpose, the keratinocytes cultured in Example 1 were treated with a trypsin-based enzyme, and then the cells were harvested and centrifuged at 1000 rpm for 5 minutes. Then, the supernatant was discarded, the cells were well resuspended by adding the condition-based culture medium shown in Table 2 below, and then the cell numbers were measured using NC-250™. The cells were dispensed at $1 \times 10^4$ cells per well in a 6-well plate, and incubated in a 37°C incubator for 4 days, and a photograph of the cells taken on the 4th day of culture is shown in FIG. 2a. Subsequently, the cells were treated with a trypsin-based enzyme on the 4th day of culture, and then the cells were harvested and centrifuged at 1000 rpm for 5 minutes. The supernatant of the centrifuged cells was discarded, and the cells were well resuspended by adding the condition-based culture medium shown in Table 2 below, and then the cell numbers were measured using NC-250™. The results are shown in FIG. 2b.

As a result, as shown in Table 2, FIGS. 2a and 2b, it was confirmed that in the case of epidermal growth factor, the cell morphology and yield become good at a concentration of 0.01 ~ 0.1ng/ml, and in the case of albumin, they become good at a concentration of 10 ~ 100ug/ml. Accordingly, it was found that the optimal effective concentrations of epidermal growth factor and albumin were 0.01~ 0.1ng/ml and 10 ~100ug/ml, respectively.

[Table 2]

| Group | |
|---|---|
| Albumin (ug/ml) | Epidermal growth factor (ng/ml) |
| 0 | 0, 0.01, 0.1, 1, 10 |
| 10 | 0, 0.01, 0.1, 1, 10 |
| 100 | 0, 0.01, 0.1, 1, 10 |
| 1000 | 0, 0.01, 0.1, 1, 10 |

### Example 4. Confirmation of establishment of mouse skin tissue-derived keratinocytes in a mixed medium to which component ratios of basal medium, calcium, epidermal growth factor, and albumin were applied

Based on the results of Examples 2 and 3, the present inventors conducted an experiment using LONZA's KGM-Gold media, which is an already used product, as a positive control group, to confirm the cell establishment performance of the mixed medium to which the component ratios of basic medium, calcium, epidermal growth factor, and albumin were applied. In this example, a mixed medium having a component ratio of 0.07 mM calcium, 100 ug/ml albumin, and 0.1 ng/ml epidermal growth factor was used as the basal medium.

For this purpose, the mouse tail skin tissue was isolated, the isolated tail tissue was placed in saline or DMEM (Dulbecco Modified Eagle Medium) medium and transported to the laboratory. The process of washing the tail tissue twice with sterile physiological saline in a clean bench, placing the washed tail tissue in a Petri dish, and peeling the skin of the tail was proceeded. More specifically, first, the middle from the bottom to the end of the tail was cut out using a blade, and the skin was cut out to a length of about 2~3 cm along the middle line. Then, the cut skin tissue was placed in a 100π Petri dish, and 15 ml of PBS was added to proceed with rinsing. Each skin was placed in a 2ml tube and filled with cold dispaseII digestion buffer (4 mg/ml, basal medium of each medium). The tube containing the tissue was then placed on the rotator, and incubated overnight (12~18h) at 4°C. After incubation, the DispaseII solution and each skin tissue were transferred together to a 100π Petri dish, and the remaining dispaseII was washed out with 15 ml of PBS. Then, the skin was grasped using forceps, and transferred to a new Petri dish with the epidermis facing down and the dermis facing up, and then the skin was carefully stretched completely, and the cells were isolated using a trypsin-based enzyme. After crushing the cell clumps using a pipette, the cells were filtered using a 100 $\mu$m cell strain filter, placed in a 50 ml conical tube, and centrifuged at 180 xg for 5 minutes with the 100 $\mu$m cell strain filter being fixed. The supernatant was removed by aspiration, and then the cell pellet was resuspended using cold KC culture medium, and the cell numbers were measured using NC-250™. Next, cells were dispensed at $1 \times 10^4$ per well using each medium in a collagen-coated 6-well plate, and incubated in a 37°C incubator for 5 days. Pictures taken on the first and fifth days of culture are shown in FIG. 3a. On the 5th day of culture, the cells were harvested by treatment with a trypsin-based enzyme, centrifuged at 1000 rpm for 5 minutes, the supernatant was discarded, and the cells were well resuspended by adding the condition-based culture media shown in Table 2. The cell number, cell size and viability were analyzed using NC-250™ and shown in FIG. 3b.

As a result of the experiment, as shown in FIGS. 3a and 3b, it was found that when cultured using the mixed medium of the present invention, it shows a cell morphology similar to the already used culture medium (LONZA KGM) used as a positive control, and can obtain more cell numbers.

### Example 5. Verification of the growth of keratinocytes derived from human skin tissue in a mixed medium to which component ratios of basal medium, calcium, epidermal growth factor, and albumin were applied

In order to confirm the growth ability of human skin tissue-derived keratinocytes by a mixed medium to which a component ratio of basic medium, calcium, epidermal growth factor, and albumin were applied, the present inventors used LONZA's KGM-Gold media, which is an already used product, as a positive control group, to proceed with subculture from passage 1 to passage 5. The mixed medium of the present invention used in this Example was a medium having the same composition as in Example 4.

Specifically, the cryopreserved cells were thawed and then centrifuged to remove the supernatant. The cell precipitate was suspended with each medium, and inoculated in a 25T flask coated with collagen typeIV at a concentration of $7.5 \times 10^4$/flask, and then cultured under the conditions of 37°C and 5% $CO_2$. During the culture, each medium was replaced once every 2 or 3 days. The passage proceeded each time the density of cells proliferated to 80% or more of the culture area. During passage, each cell was detached from the bottom of the flask by treating with a trypsin-based enzyme, and then the cells were centrifuged. The supernatant was discarded, the remaining cells was well resuspended by adding the condition-based medium, and the cell numbers were measured using NC-250™. The passage after measurement was performed in the same way as the cell thawing process. The cells were subjected to subculture until passage 5, and a photograph taken at cells (passage 5) is shown in FIG. 4a. In addition, using the number of cells obtained in each passage, the population doubling time (PDT), cell size and viability were analyzed and shown in FIG. 4b. The PDT of the cells was calculated according to the following Equation using the cell number and the culture time measured during the passage.

[Equation 1]

$$\text{PDT (Population Doubling time)} = \ln(2) \times (\Delta \text{time between B and A}) / \ln (\text{count B/ count A})$$

A = previous passage, B = current passage

As a result of the experiment, the morphology of the cells showed a pebble shape, which is characteristic of keratinocytes, similar to that of the already used medium (LONZA KGM) as a positive control group. In addition, the PDT of the cells was more stable in the mixed medium of the present invention than in the positive control medium while the culture progresses until passage 5. It was also confirmed that cell size and viability remained stable.

**Example 6. Confirmation of characteristics of keratinocytes in a mixed medium to which component ratios of basal medium, calcium, epidermal growth factor, and albumin were applied**

The present inventors attempted to verify the characteristics of keratinocytes cultured in the mixed medium according to the present invention using the cells cultured in Example 5.

First, the expression level of the keratinocyte marker protein was confirmed through a Western blot. After the cells cultured in Example 5 was treated with a trypsin-based enzyme, the cells were harvested, and centrifuged at 4000 rpm for 5 minutes, and proteins were isolated using RIPA solution (Biosesang, Korea). The isolated proteins were quantified using a protein quantification kit (Thermo Fisher Scientific, USA). Equal amounts of each protein were then placed in the wells of a protein gel (Invitrogen, USA), and then SDS-polyacrylamide gel electrophoresis (SDS-PAGE) was performed at 80V and 100V. Next, the protein was transferred to a membrane using a membrane transfer kit (Invitrogen, USA), and then primary and secondary antibodies were sequentially added and allowed to react. After completion of the reaction, the protein expression level was confirmed using HRP-ECL solution (Thermo Fisher Scientific, USA) and iBright (Thermo Fisher Scientific, USA), and GAPDH (glyceraldehyde-3-phosphate dehydrogenase) protein was used as a control group.

As a result, as shown in FIG. 5, it was confirmed that when cultured with the mixed medium of the present invention, the expression of Cytokeratin 5 and Cytokeratin 14, which are early expression markers of keratinocytes, is identical or increased as compared to cells cultured with an already used medium (LONZA KGM).

In addition, in order to compare the mRNA levels of keratinocyte-specific markers through PCR, the cells cultured in Example 5 were treated with a trypsin-based enzyme, then the cells were harvested, centrifuged at 4000 rpm for 5 minutes, and RNA was isolated using easy-BLUE (Intron, Korea). Then, cDNA was synthesized from the RNA using a reverse transcription kit (Promega, USA), which was amplified using a SimpliAmp Thermal Cycler (Biosystems, Korea). In order to confirm the quantitative difference of each PCR product, a 1% agarose gel was made with 1X TAE buffer, PCR products corresponding to each primer were placed in each well, followed by electrophoresis at 100V. The gel after DNA isolation by electrophoresis was observed under iBright (Thermo fisher scientific, USA) to confirm the expression level, and 18s ribosomal RNA was used as a control group.

As a result, as shown in FIG. 6, it was confirmed that Cytokeratin 5, Cytokeratin 14, and E-cadherin were expressed similarly to the cells cultured with the already used medium (LONZA KGM). Such results demonstrate that the cells grown in the mixed medium according to the present invention stably retain cell characteristics during passage.

**Example 7, Confirmation of the activity of keratinocytes in the mixed medium to which component ratios of basal medium, calcium, epidermal growth factor, and albumin were applied**

Finally, the present inventors attempted to confirm the activity of keratinocytes cultured in the mixed medium according to the present invention using the cells cultured in Example 5. For this purpose, the cells cultured in Example 5 were treated with a trypsin-based enzyme, and then the cells were harvested and centrifuged at 1000 rpm for 5 minutes. The supernatant of the centrifuged cells was discarded, and the cells were well resuspended by adding a condition-based medium, and then the cell numbers were measured using NC-250™. Subsequently, $5 \times 10^2$ cells per well were seeded in a 6-well plate and incubated in a 37°C incubator for 7 days. To visually confirm the number and size of cell colonies generated for a certain period of time after 7 days, the culture medium was removed, and the cells were fixed with 4% formalin solution at room temperature for 30 minutes and then stained with a concentrated solution of 0.1% crystal violet dye at room temperature for 30 minutes. The colonies formed of 50 or more cells were grouped as one, and the number of colonies formed per well was analyzed.

As a result, as shown in FIG. 7, it was confirmed that the colony size of cells cultured in the mixed medium conditions of the present invention was generally larger and more colonies were formed as compared to cells cultured in the already used medium (LONZA KGM). This means that cell activity is further increased in the mixed medium to which the component ratios of basal medium, calcium, epidermal growth factor (EGF) and albumin are applied.

It is understood that the above-described embodiments are only illustrative of the application of the principles of the present invention. The present invention may be embodied in other specific forms without departing from its spirit or essential characteristics. The described embodiment is to be considered in all respects only as illustrative and not restrictive.

**Claims**

1. A culture medium additive for establishing or culturing keratinocytes, comprising calcium, an epidermal growth factor, and albumin.

**2.** The culture medium additive according to claim 1, wherein:
the keratinocytes are keratinocytes derived from human skin.

**3.** The culture medium additive according to claim 1, wherein:
the calcium is contained at a concentration of 0.03 to 0.1 mM.

**4.** The culture medium additive according to claim 1, wherein:
the epidermal growth factor is contained at a concentration of 0.001 to 1 ng/ml.

**5.** The culture medium additive according to claim 1, wherein:
the albumin is contained at a concentration of 0.001 mg/ml or more and less than 1 mg/ml.

**6.** The culture medium additive according to claim 1, wherein:
the culture medium additive is for proliferation.

**7.** A culture medium composition for establishing or culturing keratinocytes, comprising the culture medium additive of claim 1 and a basal medium.

**8.** The culture medium composition according to claim 7, wherein:
the basal medium is any one selected from the group consisting of DMEM (Dulbecco's Modified Eagle's Medium); MEM (Minimal Essential Medium); BME (Basal Medium Eagle); RPMI 1640; DMEM/F-10 (Dulbecco's Modified Eagle's Medium: Nutrient Mixture F-10); DMEM/F-12 (Dulbecco's Modified Eagle's Medium: Nutrient Mixture F-12); a-MEM (a-Minimal essential Medium); G-MEM (Glasgow's Minimal Essential Medium); IMDM (Isocove's Modified Dulbecco's Medium); KnockOut DMEM (GIBCO, USA); and MCDB.

**9.** The culture medium composition according to claim 7, wherein:
the culture medium composition suppresses the extension of population doubling time (PDT) of keratinocytes for at least 5 passages.

**10.** A method of culturing keratinocytes, comprising culturing keratinocytes in the culture medium composition of claim 7.

【FIG. 1a】

Gruop1

Gruop2

Gruop3

Gruop4

【FIG. 1b】

【FIG. 2a】

【FIG. 2b】

harvest cell number (n)

【FIG. 3a】

on the 1st day

on the 5th day

【FIG. 3b】

【FIG. 4a】

【FIG. 4b】

【FIG. 5】

GAPDH : 37kDa  Cytokeratin 5: 63kDa  Cytokeratin 14 : 55kDa   Cytokeratin 1 : 67kDa  Cytokeratin 10 : 55kDa

**Early marker**      **Late marker**

【FIG. 6】

【FIG. 7】

## INTERNATIONAL SEARCH REPORT

| International application No. |
|---|
| **PCT/KR2021/017263** |

### A.    CLASSIFICATION OF SUBJECT MATTER

**C12N 5/071**(2010.01)i

According to International Patent Classification (IPC) or to both national classification and IPC

### B.    FIELDS SEARCHED

Minimum documentation searched (classification system followed by classification symbols)

C12N 5/071(2010.01); A61K 35/12(2006.01); A61K 38/18(2006.01); C12N 5/00(2006.01); C12N 5/02(2006.01); C12N 5/06(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 각질형성세포(keratinocyte), 칼슘(calcium), 상피세포성장인자(epidermal frowth factor), 알부민(albumin), 배지(medium), 첨가제(additive)

### C.    DOCUMENTS CONSIDERED TO BE RELEVANT

| Category* | Citation of document, with indication, where appropriate, of the relevant passages | Relevant to claim No. |
|---|---|---|
| Y | US 5712163 A (PARENTEAU, N. L. et al.) 27 January 1998 (1998-01-27)<br>    See column 17, lines 54-66; column 22, lines 30-38; and claims 1, 4, 5 and 24. | 1-10 |
| Y | WO 2006-113629 A1 (THE TRUSTEES OF COLUMBIA UNIVERSITY IN THE CITY OF NEW YORK) 26 October 2006 (2006-10-26)<br>    See claims 6, 8, 10 and 12. | 1-10 |
| Y | US 2002-0164793 A1 (CONRAD, P. B. et al.) 07 November 2002 (2002-11-07)<br>    See abstract; and claims 50 and 54. | 1-10 |
| A | KR 10-0455006 B1 (SOCIETE DES PRODUITS NESTLE S.A.) 13 January 2005 (2005-01-13)<br>    See entire document. | 1-10 |
| A | KR 10-2003-0043313 A (HANS BIOMED CORP) 02 June 2003 (2003-06-02)<br>    See entire document. | 1-10 |

☐ Further documents are listed in the continuation of Box C.        ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **22 February 2022** | **22 February 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

| Patent document cited in search report | | | Publication date (day/month/year) | Patent family member(s) | | | Publication date (day/month/year) |
|---|---|---|---|---|---|---|---|
| US | 5712163 | A | 27 January 1998 | EP | 0403139 | A1 | 19 December 1990 |
| | | | | EP | 0403139 | B1 | 12 August 1998 |
| | | | | JP | 03-087174 | A | 11 April 1991 |
| | | | | JP | 2577114 | B2 | 29 January 1997 |
| WO | 2006-113629 | A1 | 26 October 2006 | EP | 1937798 | A1 | 02 July 2008 |
| | | | | US | 2008-0261259 | A1 | 23 October 2008 |
| | | | | US | 2008-0268490 | A1 | 30 October 2008 |
| US | 2002-0164793 | A1 | 07 November 2002 | AU | 2002-255618 | B2 | 07 September 2006 |
| | | | | AU | 2006-249240 | A1 | 04 January 2007 |
| | | | | AU | 2006-249240 | B2 | 27 May 2010 |
| | | | | AU | 2010-214639 | A1 | 16 September 2010 |
| | | | | AU | 2010-214639 | B2 | 19 April 2012 |
| | | | | CA | 2439899 | A1 | 12 September 2002 |
| | | | | CA | 2439899 | C | 29 September 2015 |
| | | | | DK | 1404809 | T3 | 17 March 2014 |
| | | | | EP | 1404809 | A2 | 07 April 2004 |
| | | | | EP | 1404809 | B1 | 11 December 2013 |
| | | | | ES | 2457092 | T3 | 24 April 2014 |
| | | | | IL | 157667 | A | 22 September 2009 |
| | | | | IL | 195076 | A | 29 February 2012 |
| | | | | JP | 2005-505312 | A | 24 February 2005 |
| | | | | JP | 2009-195232 | A | 03 September 2009 |
| | | | | JP | 4317366 | B2 | 19 August 2009 |
| | | | | JP | 4989674 | B2 | 01 August 2012 |
| | | | | NZ | 528166 | A | 31 May 2007 |
| | | | | NZ | 549526 | A | 26 June 2009 |
| | | | | NZ | 549527 | A | 30 April 2008 |
| | | | | NZ | 549528 | A | 30 April 2008 |
| | | | | PT | 1404809 | E | 20 March 2014 |
| | | | | US | 2002-0168768 | A1 | 14 November 2002 |
| | | | | US | 2002-0187498 | A1 | 12 December 2002 |
| | | | | US | 2005-0226853 | A1 | 13 October 2005 |
| | | | | US | 2006-0121608 | A1 | 08 June 2006 |
| | | | | US | 2010-0099576 | A1 | 22 April 2010 |
| | | | | US | 6846675 | B2 | 25 January 2005 |
| | | | | US | 6974697 | B2 | 13 December 2005 |
| | | | | US | 7407805 | B2 | 05 August 2008 |
| | | | | US | 7501238 | B2 | 10 March 2009 |
| | | | | US | 7541188 | B2 | 02 June 2009 |
| | | | | US | 7955790 | B2 | 07 June 2011 |
| | | | | WO | 02-070729 | A2 | 12 September 2002 |
| | | | | WO | 2002-070729 | A3 | 22 January 2004 |
| KR | 10-0455006 | B1 | 13 January 2005 | AT | 199390 | T | 15 March 2001 |
| | | | | AU | 1305497 | A | 17 July 1997 |
| | | | | AU | 730222 | B2 | 01 March 2001 |
| | | | | BR | 9612256 | A | 13 July 1999 |
| | | | | BR | 9612256 | B1 | 10 August 2010 |
| | | | | CA | 2234118 | A1 | 03 July 1997 |
| | | | | CA | 2234118 | C | 07 April 2009 |
| | | | | CN | 1154717 | C | 23 June 2004 |

Form PCT/ISA/210 (patent family annex) (July 2019)

<table>
<tr><td colspan="2"><strong>INTERNATIONAL SEARCH REPORT</strong><br>Information on patent family members</td><td colspan="2">International application No.<br><strong>PCT/KR2021/017263</strong></td></tr>
<tr><td>Patent document<br>cited in search report</td><td>Publication date<br>(day/month/year)</td><td>Patent family member(s)</td><td>Publication date<br>(day/month/year)</td></tr>
<tr><td></td><td></td><td>CN   1205737   A</td><td>20 January 1999</td></tr>
<tr><td></td><td></td><td>CZ   194598   A3</td><td>11 November 1998</td></tr>
<tr><td></td><td></td><td>CZ   297289   B6</td><td>11 October 2006</td></tr>
<tr><td></td><td></td><td>CZ   9801945   A3</td><td>11 November 1998</td></tr>
<tr><td></td><td></td><td>DE   69611894   T2</td><td>13 September 2001</td></tr>
<tr><td></td><td></td><td>DK   0780469   T3</td><td>26 March 2001</td></tr>
<tr><td></td><td></td><td>EP   0780469   A1</td><td>25 June 1997</td></tr>
<tr><td></td><td></td><td>EP   0780469   B1</td><td>28 February 2001</td></tr>
<tr><td></td><td></td><td>EP   0877797   A1</td><td>18 November 1998</td></tr>
<tr><td></td><td></td><td>ES   2155166   T3</td><td>01 May 2001</td></tr>
<tr><td></td><td></td><td>GR   3035719   T3</td><td>31 July 2001</td></tr>
<tr><td></td><td></td><td>HU   226195   B1</td><td>30 June 2008</td></tr>
<tr><td></td><td></td><td>HU   9903742   A2</td><td>28 March 2000</td></tr>
<tr><td></td><td></td><td>HU   9903742   A3</td><td>30 October 2000</td></tr>
<tr><td></td><td></td><td>IL   124191   A</td><td>01 June 2004</td></tr>
<tr><td></td><td></td><td>JP   2000-506374   A</td><td>30 May 2000</td></tr>
<tr><td></td><td></td><td>JP   4404965   B2</td><td>27 January 2010</td></tr>
<tr><td></td><td></td><td>NO   326190   B1</td><td>13 October 2008</td></tr>
<tr><td></td><td></td><td>NO   982810   A</td><td>21 August 1998</td></tr>
<tr><td></td><td></td><td>NO   982810   D0</td><td>18 June 1998</td></tr>
<tr><td></td><td></td><td>NZ   325814   A</td><td>27 October 2000</td></tr>
<tr><td></td><td></td><td>PL   195108   B1</td><td>31 August 2007</td></tr>
<tr><td></td><td></td><td>PL   327320   A1</td><td>07 December 1998</td></tr>
<tr><td></td><td></td><td>PT   780469   E</td><td>29 June 2001</td></tr>
<tr><td></td><td></td><td>RU   2215030   C2</td><td>27 October 2003</td></tr>
<tr><td></td><td></td><td>SK   283314   B6</td><td>02 May 2003</td></tr>
<tr><td></td><td></td><td>SK   83598   A3</td><td>11 January 1999</td></tr>
<tr><td></td><td></td><td>US   2002-0012993   A1</td><td>31 January 2002</td></tr>
<tr><td></td><td></td><td>US   2002-0042129   A1</td><td>11 April 2002</td></tr>
<tr><td></td><td></td><td>US   6423540   B2</td><td>23 July 2002</td></tr>
<tr><td></td><td></td><td>US   6949381   B2</td><td>27 September 2005</td></tr>
<tr><td></td><td></td><td>WO   97-23602   A1</td><td>03 July 1997</td></tr>
<tr><td>KR   10-2003-0043313   A</td><td>02 June 2003</td><td>None</td><td></td></tr>
</table>

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Expert Rev Endocrinol Metab.,* July 2012, vol. 7 (4), 461-472